# EUROPEAN PATENT APPLICATION

(11) **EP 2 601 929 A1**
(43) Date of publication of application: **12.06.2013**
(21) Application number: 11814271.0
(22) Date of filing: 29.07.2011
(51) Int. Cl.: A61K 8/40, A61K 8/36, A61K 8/41, A61K 8/46, A61Q 5/12

(54) **HAIR CARE PRODUCT**

(30) Priority: 03.08.2010 JP 2010174307
(71) Applicant: Kao Corporation, Chuo-Ku Tokyo 103-8210 (JP)
(72) Inventor: TETSU, Makio, Tokyo 131-8501 (JP); MORIOKA, Tomoki, Tokyo 131-8501 (JP)
(74) Representative: HOFFMANN EITLE
(86) International application number: PCT/JP2011/004320
(87) International publication number: WO 2012/017634

(57) **Abstract**

The present invention presents a hair cosmetic composition, which contains (A) equal to or higher than 0.01 mass % and equal to or lower than 20 mass % of a quaternary ammonium or its salt; (B) equal to or higher than 0.01 mass % and equal to or lower than to 20 mass % of ether-type tertiary amine or its salt; (C) equal to or higher than 0.005 mass % and equal to or lower than 6 mass % of branched aliphatic acid or its salt; and (D) equal to or higher than 0.05 mass % and equal to or lower than 10 mass % of aromatic sulfonate and water, in which the mass ratio of the component (C) and the component (D) being (C)/(D) is 0.05 to 1.

## Description

### Field of the Invention

The present invention relates to a hair cosmetic composition. More specifically, the present invention relates to a conditioner, which provides good feel after drying.

### Background of the Invention

Technologies for providing smoothness and moist feel to the hair have been conventionally employed for hair cosmetics, in particular a conditioner by directly modifying the properties of hair surface. For example, Patent Document 1 discloses that the use of a particular compound such as 2-naphthalenesulfonic acid and the like, together with the use of a cationic surfactant, provides a hair cosmetic composition, which achieves enhanced manageability for the hairstyle.

On the contrary, an enhanced voluminous look of the hair is known as an effect opposite to the manageability of hair. In Patent Documents 2 and 3, particles are mixed to a hair care composition to cause adhesion on the surface of the hair, so as to maintain the voluminous hairstyle. In addition, Patent Document 4 discloses a technology for obtaining voluminous look of the hair by employing latex particles adsorbed on keratin fibers. Related Documents

### Patent Documents

### [Patent Document 1]

Japanese Laid-open patent publication No. H11-228,358

### [Patent Document 2]

Japanese Patent Domestic Publication for PCT Application No. 2005-511,583

### [Patent Document 3]

Japanese Patent Domestic Publication for PCT Application No. 2005-506,312

### [Patent Document 4]

Japanese Laid-open patent publication No. 2004-137,274 Summary of the Invention

In recent years, it is required to obtain a manageable hairstyle while maintaining voluminous look of the hair around the top of the head. However, such problem was not able to be solved by adopting any of the technologies described in Patent Documents 1 to 4. In addition, when particles or the like are mixed in a conditioner, the hair after drying tends to creak, failing to obtain better finish texture. Further, the particles are stuck to the scalp surface, causing a problem of insufficient rising of the hair root at the top of the head.

The present inventors eagerly investigated hair cosmetic compositions which provide manageable hairstyle while achieving voluminous look of the hair around the top of the head. It is eventually found that a hair composition having manageability of the hairstyle is obtained while enhancing the rising of the hair roots around the top of the head, with a hair composition combining components of a quaternary ammonium or its salt, ether-type tertiary amine or its salt, branched aliphatic acid or its salt, and aromatic sulfonate.

According to the present invention, there is provided a hair cosmetic composition containing:
(A) equal to or higher than 0.01 mass % and equal to or lower than 20 mass % of a quaternary ammonium represented by the following formula (1) or its salt;

   R¹R²R³R⁴N⁺X⁻ (1)

   [In formula (1), at least one of R¹, R², R³ and R⁴ represents a linear or branched alkyl group or alkenyl group having 16 to 25 carbon atoms, which may have ether bond and at least two or more of the rest thereof represent a same or different alkyl group having 1 to 6 carbon atoms or -(AO)ₘH (A represents a same or different alkylene group having 2 to 4 carbon atoms, m represents an integer number of 1 to 6, and the sequence is arbitrary) and X⁻represents a halide ion or alkyl sulfate ion having 1 to 2 carbon atoms.]
(B) equal to or higher than 0.01 mass % and equal to or lower than 20 mass % of ether-type tertiary amine, represented by the following formula (2) or its salt;

   R⁹- (O-R²⁴)ₑ-NR¹⁰R¹¹ (2)

   [In formula (2), R⁹ represents a linear or branched alkyl or alkenyl group having 6 to 24 carbon atoms, R¹⁰ and R¹¹ represent a same or different alkyl group having 1 to 6 carbon atoms or - (AO)ₘH (A represents a same or different alkylene group having 2 to 4 carbon atoms, m represents an integer number of 1 to 6, and the sequence is arbitrary), and R²⁴ represents a linear alkyl group having 1 to 3 carbon atoms, which may be substituted with a hydroxy group (e represents an integer number of 1 to 5).]
(C) equal to or higher than 0.005 mass % and equal to or lower than 6 mass % of branched aliphatic acid represented by the following formula (3) or its salt;

   CH₃-CHR¹⁸- (CH₂)ₙCOOH (3)

   [In formula (3), R¹⁸ represents a methyl group or ethyl group and n represents an integer number of 14 to 20.]
(D) equal to or higher than 0.05 mass % and equal to or lower than 10 mass % of aromatic sulfonate and water, wherein the mass ratio of the component (C) and the component (D) being (C)/(D) is 0.05 to 1.

In addition, the present invention provides a use of the above-described hair cosmetic composition, which is applied to the hair and then is rinsed with water to provide conditioning effect to the hair.

In addition, the present invention provides a use of the above-described hair cosmetic composition for producing a composition, which is applied to the hair and then is rinsed with water to provide conditioning effect to the hair.

Further, the present invention provides a method, in which the above-described hair cosmetic composition is applied to the hair and then is rinsed with water.

According to the present invention, there is obtained the hair cosmetic composition for obtaining manageable hairstyle while maintaining voluminous look around the top of the head.

### Brief Description of the Drawings

The present invention will be more apparent from the following description of certain preferred embodiments taken in conjunction with the following accompanying drawings.

### [Fig. 1]

It is a cross-sectional view, which schematically represents a cross section of a model hair bundle 10.

### [Fig. 2]

It is a schematic diagram, which is useful for describing a method for measuring the rising level.

### [Fig. 3]

It is a schematic diagram, which is useful for describing a method for measuring the rising level.

### [Fig. 4]

It is a schematic diagram, which is useful for describing a method for measuring the rising level.

### Detailed Description of the Invention

### [Component (A): quaternary ammoniums or their salts]

A cationic surfactant of component (A) of the present invention is represented by the following formula (1).

R¹R²R³R⁴N⁺X⁻ (1)

[In formula (1), at least one of R¹, R², R³ and R⁴ represents a linear or branched alkyl group or alkenyl group having 16 to 25 carbon atoms, which may have ether bond, at least two or more of the rest thereof represent a same or different alkyl group having 1 to 6 carbon atoms or -(AO)ₘH (A represents a same or different alkylene group having 2 to 4 carbon atoms, m represents an integer number of 1 to 6, and the sequence is arbitrary) and X⁻ represents a halide ion or alkyl sulfate ion having 1 to 2 carbon atoms.]

The salt of the quaternary ammonium represented by the formula (1) may be obtained by employing a salt of a quaternary ammonium prepared with an organic acid, an inorganic acid, or both, or may be obtained by creating a salt in the composition together with conducting pH control by blending an acid in the hair cosmetic composition of the present invention.

The typical organic acid includes, for example, an acid having short chain alkyl group such as alkylphosphate, alkyl sulfonic acid, alkyl sulfate and the like; acidic amino acid such as L-glutamic acid, L-aspartic acid and the like; pyroglutamic acid; aromatic acid such as benzoic acid, p-toluenesulfonic acid and the like; hydroxy acid such as glycolic acid, lactic acid, glyceric acid, gluconic acid, pantothenic acid, malic acid, tartaric acid, citric acid and the like. Inorganic acid includes phosphoric acid, hydrochloric acid, acetic acid, succinic acid and the like. Among these, organic acids are preferable, in view of achieving moisture retention and softening effect for the hair, and acidic amino acid, pyroglutamic acid, hydroxy acid are more preferable, and hydroxy acid is even more preferable.

Specific examples of the component (A) include the following (A-i) to (A-iii).

(A-i) It typically includes alkyltrimethylammonium salt, for example compounds represented by the following general formula (4):

R⁵-N⁺(CH₃)₃X⁻ (4)

[In the formula, R⁵ represents a linear or branched alkyl group having 16 to 22 carbon atoms and X⁻ represents a halide (chlorine or bromine) ion or alkyl sulfate ion having 1 to 2 carbon atoms]

(A-ii) It typically includes alkoxy trimethylammonium salt, for example compound represented by the following general formula (5):

R⁶-O-R⁷-N⁺(CH₃)₃X⁻ (5)

[In the formula, R⁶ represents a linear or branched alkyl group having 16 to 22 carbon atoms, R⁷ represents an ethylene group or propylene group and X⁻ represents a halide (chlorine or bromine) ion or alkyl sulfate ion having 1 to 2 carbon atoms]

(A-iii) It typically includes dialkyl dimethylammonium salt, for example compound represented by the following general formula:

R⁸₂-N⁺(CH₃)₂X⁻ (6)

[In the formula, R⁸ represents a linear or branched alkyl group having 16 to 22 carbon atoms and X⁻ is the same as described above.]

The typical cationic surfactants except the above-described (A-i) to (A-iii) include lanolin fatty acid aminopropylethyldimethylammonium ethylsulfate (ethyl sulfate of alkanoyl aminopropyl dimethylethyl ammonium, alkanoyl group is derived from lanolin), lanolin fatty acid aminoethyltriethylammonium ethylsulfate; lanolin fatty acid aminopropyltriethylammonium ethylsulfate; lanolin fatty acid aminoethyltrimethylammonium methylsulfate; lanolin fatty acid aminopropylethyldimethylammonium methylsulfate; isoalkanoic acid (14 to 20 carbon atoms) aminopropylethyldimethylammonium ethylsulfate; isoalkanoic acid (18 to 22 carbon atoms) aminopropylethyldimethylammonium ethylsulfate; isostearic acid aminopropylethyldimethylammonium ethylsulfate; isononanoic acid aminopropylethyldimethylammonium ethylsulfate; alkyltrimethylarmonium saccharin and the like. Among these, it is preferable to use (A-i) alkyl trimethylammonium (or their salts), in view of effects for providing enhanced softness and smoothness, and natural texture of the finished hair obtained by the use thereof, and among these, trimethylammonium (or its salt) having an alkyl group having 16 to 22 carbon atoms is preferable, and trimethylammonium (or its salt) having an alkyl group having 16 to 18 carbon atoms is more preferable, and further cetyltrimethylammonium salt is even more preferable.

One type of, or two or more types of cationic surfactant(s) may be used in combination with the component (A).

The content of the component (A) is, in view of effects for providing enhanced softness and smoothness, and natural texture of the finished hair obtained by the use thereof, equal to or higher than 0.01 mass % over the whole composition, and more preferably equal to or higher than 0.1 mass %, and even more preferably equal to or higher than 0.5 mass %. On the other hand, the content is, in view of effects for providing enhanced softness and smoothness, and natural texture of the finished hair obtained by the use thereof, equal to or lower than 20 mass % over the whole composition, more preferably equal to or lower than 10 mass %, and even more preferably equal to or lower than 5 mass %.

It is preferable to further contain at least equal to or higher than 0.3 mass % of cetyltrimethylammonium chloride in the component (A). It is preferable to use a mixture of two types of compounds, which are cetyltrimethylammonium chloride and other type of quaternary ammonium salt. More specifically, a combination of cetyltrimethylammonium chloride and stearyltrimethylammonium chloride is preferable.

### [Component (B): ether-type tertiary amines or their salts]

The component (B) of the present invention is represented by the following formula (2).

R⁹- (O-R²⁴)ₑ-NR¹⁰R¹¹ (2)

[In formula (2), R⁹ represents a linear or branched alkyl group or alkenyl group having 6 to 24 carbon atoms, R¹⁰ and R¹¹ represent a same or different alkyl group having 1 to 6 carbon atoms or - (AO)ₘH (A represents a same or different alkylene group having 2 to 4 carbon atoms, m represents an integer number of 1 to 6, and the sequence is arbitrary), and R²⁴ represents a linear alkyl group having 1 to 3 carbon atoms, which may be substituted with hydroxy group (e represents an integer number of 1 to 5).]

R⁹ is preferably the linear or branched alkyl group or alkenyl group having 12 to 24 carbon atoms, more preferably 14 to 22 carbon atoms, and alkyl group having such number of carbon atoms is even more preferable. Further, it is preferable that at least one of R¹⁰ and R¹¹ is an alkyl group having 1 to 6 carbon atoms. Among these, methyl group or ethyl group is preferable, and it is even more preferable that both are the same.

A salt of a tertiary amine represented by the general formula (2) may be formed by means of an organic acid, an inorganic acid, or both, or may be formed in the composition at the same time when an acid may be formulated in the hair composition as adjustment of the pH.

The typical organic acids include, for example, acids having a short chain alkyl group such as alkyl phosphoric acid, alkyl sulfonic acid, alkyl sulfuric acid and the like; acidic amino acids such as L-glutamic acid, L-aspartic acid and the like; pyroglutamic acid; aromatic acids such as benzoic acid, p-toluenesulfonic acid and the like; hydroxy acids such as glycolic acid, lactic acid, glyceric acid, gluconic acid, pantothenic acid, malic acid, tartaric acid, citric acid and the like, and typical inorganic acids include phosphoric acid, hydrochloric acid, acetic acid, succinic acid and the like. Among these, in view of providing moisture retention and softening effect for the hair, organic acids are preferable, and acidic amino acids, pyroglutamic acid and hydroxy acid are more preferable, and hydroxy acid is even more preferable.

More specific examples of the cationic surfactants for the component (B) includes, for example, at least one of the following (B-i) to (B-ii) tertiary amine compounds (or their salts) and the like.

### (B-i) Hydroxyether alkyl amines (or their salts)

For example, compounds represented by the following general formula (7) and their salts are exemplified.

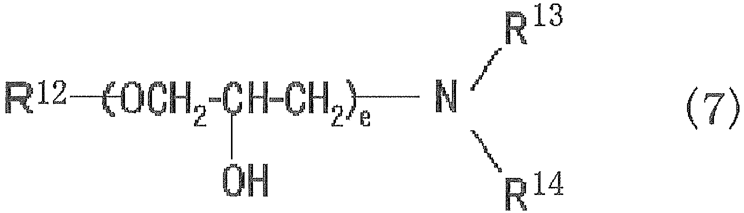

[In formula, R¹² represents a linear or branched alkyl group or alkenyl group having 6 to 24 carbon atoms; R¹³ and R¹⁴ represent a same or different alkyl group having 1 to 6 carbon atoms or - (AO)_{f}H (A represents an alkylene group having 2 to 4 carbon atoms, f represents a same or different integer number of 1 to 6, and the sequence is arbitrary), and e represents an integer number of 1 to 5).]

More specifically, hexadecyloxy(2-hydroxypropyl)dimethylamine and its salt, octadecyloxy(2-hydroxypropyl)dimethylamine and its salt, are exemplified.

### (B-ii) Ether amines (or their salts)

For example, compounds represented by the following general formula (8) and their salts are exemplified.

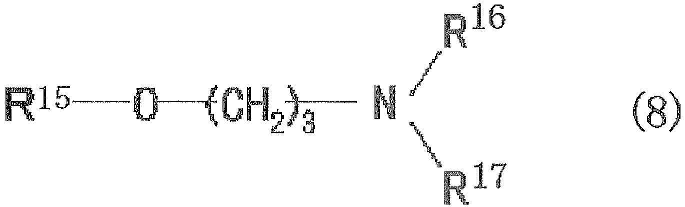

[In formula, R¹⁵ represents a linear or branched alkyl group or alkenyl group having 6 to 24 carbon atoms; R¹⁶ and R¹⁷ represent a same or different alkyl group having 1 to 6 carbon atoms or - (AO)_{g}H (A represents an alkylene group having 2 to 4 carbon atoms, g represents an integer number of 1 to 6, each of "g"-pieces of "A"s may be same or different, and the sequence is arbitrary).]

More specifically, N,N-dimethyl-3-hexadecyloxypropylamine and its salt, N,N-dimethyl-3-octadecyloxypropylamine and its salt are exemplified. Among these, (B-ii) ether amines (or their salts) are more preferable, and even more preferable examples of such ether amines include, N,N-dimethyl-3-hexadecyloxypropylamine, N,N-dimethyl-3-octadecyloxypropylamine.

Two or more types of ether amines or their salts of the component (B) may be used in combination.

The content of component (B) is, in view of effects for providing enhanced softness and smoothness, and natural texture of the finished hair obtained by the use thereof, equal to or higher than 0.01 mass % over the whole composition, and more preferably equal to or higher than 0.1 mass %, and even more preferably equal to or higher than 0.3 mass %. On the other hand, the content is, in view of effects for providing enhanced softness and smoothness, and natural texture of the finished hair obtained by the use thereof, equal to or lower than 20 mass % over the whole composition, more preferably equal to or lower than 10 mass %, and even more preferably equal to or lower than 5 mass %.

### [Component (C): branched aliphatic acids or their salts]

The component (C) of the present invention is represented by the following formula (3).

CH₃-CHR¹⁸- (CH₂)ₙCOOH (3)

[In formula (3), R¹⁸ represents a methyl group or ethyl group and n represents an integer number of 14 to 20.]

The component of the present invention (C) may be separated and extracted from the hair according to, for example, the description of LIPIDS, vol. 23, No. 9, 878-881 (1988), and may alternatively be synthesized according to the Patent Document WO98/30532.

The branched aliphatic acid is represented by the general formula (3), where R¹⁸ represents a methyl group or ethyl group, and n is 14 to 20, and preferably 16 to 20. 18-methyleicosanoic acid, 14-methylpentadecanoic acid, 14-methylhexadecanoic acid, 15-methylhexadecanoic acid, 15-methylheptadecane acid, 16-methylheptadecane acid, 16-methyloctadecane acid, 17-methyloctadecane acid and 17-methylnonadecane acid are even more preferable.

In addition, salts of such branched aliphatic acids includes: alkali metal salts such as sodium salt, lithium salt, potassium salt and the like; alkaline earth metal salts such as calcium salt, magnesium salt and the like; organic amine salts such as triethanolamine salt, diethanolamine salt, monoethanolamine salt and the like; basic amino acid salts such as lysine salt, arginine salt and the like.

The typical extracted composition includes extractants from lanolin, more specifically lanolin fatty acid. Lanolin fatty acid contains methyl branched-longchain fatty acid, which is called iso-fatty acid or anteiso-fatty acid, on the order of 50 wt %. More specifically, CRODACID 18-MEA [CRODA JAPAN KK], SKLIRO [CRODA JAPAN KK] and FA-NH [NIPPON FINE CHEMICAL CO., LTD.] are exemplified.

One of component (C) may be employed, or two or more types thereof may be in combination. In addition, combination of a synthesis composition and an extracted composition may be employed.

The content of the component (C) is equal to or higher than 0.005 mass% over the whole composition, and more preferably equal to or higher than 0.01 mass %, and even more preferably equal to or higher than 0.02 mass %, in view of effects for maintaining soft touch feeling, providing voluminous look of whole hair and better rising of hair root around the top of the head, and providing a recovery from, or inhibiting of, the damage of the hair. On the other hand, the content is equal to or lower than 6 mass % over the whole composition, more preferably equal to or lower than 3 mass %, and even more preferably equal to or lower than 1 mass %, in view of effects for maintaining soft touch feeling, providing voluminous look of whole hair and better rising of hair root around the top of the head, and providing a recovery from, or inhibiting of, the damage of the hair.

### [Component (D): aromatic sulfonate]

The typical aromatic sulfonates of the component (D) of the present invention include salts of compounds having single aromatic ring such as 2-naphthalenesulfonic acid, oxybenzone sulfonic acid, salicylic acid or guaiazulene sulfonic acid or the like. Counter ion typically includes, for example, alkali metal salts such as sodium, potassium and the like, alkaline earth metal salts calcium, magnesium and the like. Sodium 2-naphthalenesulfonate is preferable.

One compound of the component (D) may be used, or two or more types thereof may be used in combination.

The content of the component (D) is preferably equal to or higher than 0.05 mass % over the whole composition, and more preferably equal to or higher than 0.1 mass %, and even more preferably equal to or higher than 0.2 mass %, in view of effects for maintaining soft touch feeling, providing voluminous look for the whole hair and better rising of hair root around the top of the head. On the other hand, the content is preferably equal to or lower than 10 mass % over the whole composition, more preferably equal to or lower than 6 mass %, and even more preferably equal to or lower than 2 mass %, in view of effects for maintaining soft touch feeling, providing voluminous look for the whole hair and better rising of hair root around the top of the head.

The mass ratio (content ratio) of the component (C) and the component (D) being (C)/(D) is preferably within a range of from 0.05 to 1, and more preferably within a range of from 0.1 to 0.5, in view of maintaining better soft touch feeling for the hair, providing voluminous look for the entire hair, better rising of hair roots around the top of the head, good feel after drying, easy subsequent styling and easy thermal shaping. The component
(A) and the component (B) may be mixed in advance to be neutralized, so that the resultant acid addition salt may be used. In addition to above, in the hair cosmetic composition of the present invention, it is estimated that the components (A), (B), (C) and (D) create a hydrophobic complex, which is easily adsorbed onto the hair surface.

The advantageous effect of the present invention will be described. The hair after being applied with a conventional hair cosmetic composition causes mutual adhesions of the hair strands, such that the hair strands in the top area of the head bend at around the roots of the strands due to the larger weight of the adhered hair, and therefore the voluminous look is easily lost in the top area of the head. On the contrary, the hair cosmetic composition of the present invention achieves the advantageous effect of obtaining manageable hairstyle while maintaining voluminous look around the top of the head by employing the combination of the above-described components (A) to (D). While the detailed mechanism is not known, the following phenomena are presumed.

First, it is considered that a complex is created among the hair strands during the drying of the hair by combining the component (A) and the component (D) of the present invention to obtain a smooth feeling and a manageable feeling. On the contrary, it is considered that a hydrophobic complex is formed on the hair surface by combining the component (C) and the component (D) of the present invention, so that a natural texture and flexibility are obtained in the finish of the treatment, and a damage of the hair surface is inhibited and is repaired. Thus, it is considered that the combination of the components (A) to (D) causes mutual actions of the hydrophobic complex on the hair surface and the complex among the hair strands, which leads to a formation of spaces among each of the strands of hair, so that the hair root can spontaneously and naturally rise. This allows obtaining manageable hairstyle while maintaining voluminous look around the top of the head. In particular, enhanced manageability in the side region of the head is obtained. In addition, the rising of the hair root around the top of the head also achieves voluminous look for the entire hair. Further advantageous effect of allowing easy thermal shaping after drying is achieved for the hair after the hair cosmetic composition of the present invention is applied.

Preferable combination of the component (A) and component (D) of the present invention may be, for example, a combination of cetyltrimethylammonium salt and sodium 2-naphthalenesulfonate.

### [Other components]

The hair cosmetic composition of the present invention may further contain aliphatic alcohols, in view of easy application, smooth feel, and soft touch. And preferable aliphatic alcohols may have alkyl group or alkenyl group having 12 to 22 carbon atoms. For example, behenyl alcohol, stearyl alcohol, cetyl alcohol, myristyl alcohol, lauryl alcohol, oleyl alcohol, isostearyl alcohol and the like are exemplified. Among these, stearyl alcohol and cetyl alcohol are preferable. One of these aliphatic alcohols may be employed alone, or a combination of several types thereof.

One of aliphatic alcohols may be employed, or two or more types thereof may be jointly employed. In view of the smooth feeling and the soft touch, the content thereof is preferably 0.05 to 20 mass % in the hair cosmetic composition of the present invention, and more preferably 1 to 10 mass %. The content of 2 to 5 mass % is even more preferable.

The quantity of water contained in the hair cosmetic composition of the present invention is preferably 30 to 95 mass %, more preferably 50 to 95 mass %, and even more preferably 60 to 90 mass %.

In order to further improve enhanced touch feel to the hair, silicones, oil-based components, aliphatic amines except the above-described (B) and their salts and the like may be added to the hair cosmetic composition of the present invention.

Typical silicones may include, for example the following compounds:
(i) Highly-polymerized dimethyl polysiloxane, for example, BY11-026, BY22-19 [Dow Corning Toray Co., Ltd], FZ-3125 [NIPPON UNICAR CO., LTD.] and the like.

The highly-polymerized dimethyl polysiloxane available in the present invention may be dissolved or dispersed in a liquid oil (for example, liquid silicone oil such as the following (ii) dimethylpolysiloxane oil, (iii) cyclic silicone and the like, or liquid hydrocarbon oil such as isoparaffin and the like).

(ii) Dimethylpolysiloxane oils represented by the following general formula (9) :

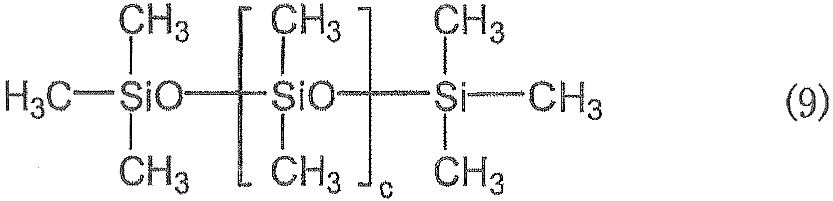

[In the formula, c represents an integer number of 0 to 650]

More specifically, commercially available compositions such as SH200C series, viscosity 1 cs, 50 cs, 200 cs, 1,000 cs, 5,000 cs [DOW CORNING TORAY CO.,LTD.] and the like may be employed.

(iii) Cyclic silicones represented by the following general formula (10):

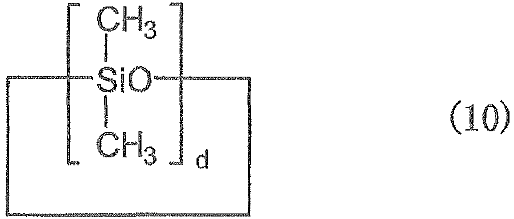

[In the formula, d represents an integer number of 3 to 7]

More specifically, dodecamethylcyclohexasiloxane, octamethylcyclotetrasiloxane, decamethylcyclopentasiloxane and the like are exemplified. In addition, the commercially available compositions include SH244 or SH245 (DOW CORNING TORAY CO.,LTD.).

(iv) Amino-modified silicone represented by the following general formula (11):

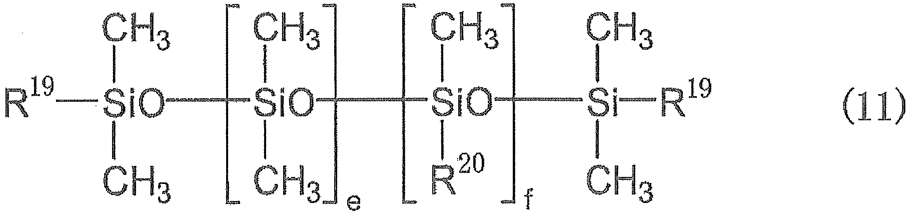

[In the formula, R¹⁹ is the same as R²⁰ or a methyl group or a hydroxyl group and R²⁰ is a reactive functional group represented by -R²¹-Q (where R²¹ represents a divalent hydrocarbon group having 3 to 6 carbon atoms, Q represents group having a primary, secondary or tertiary amino group or having an ammonium group). Each of e and f represents an integer numbers, and e+f depends on the molecular weight. Preferable average molecular weight is 3,000 to 100,000.]

For example, SS-3551, SF8452C, DC 929, DC 8500 (DOW CORNING TORAY CO.,LTD.), KT1989 (GE TOSHIBA CORPORATION) are exemplified. When the amino-modified silicone is employed as an aqueous emulsion, the quantity of the amino-modified silicone contained in the aqueous emulsion is preferably 20 to 60 wt %., and more preferably 30 to 50 wt %. Preferable amino-modified silicone aqueous emulsion includes SM8704C (DOW CORNING TORAY CO.,LTD.).

### (v) Other silicones

Available silicones except the above-described silicones include polyether-modified silicone, methylphenylpolysiloxane, fatty acid-modified silicone, alcohol-modified silicone, alkoxy-modified silicone, epoxy-modified silicone, fluorine-modified silicone, cyclic silicone, alkyl-modified silicone and the like.

The typical oil-based component includes, for example, components shown below. Higher fatty acids except the component (C) such as capric acid, lauric acid, myristic acid, palmitic acid, stearic acid, behenic acid, oleic acid, coconut oil fatty acid and the like; hydrocarbon oils such as liquid paraffin, liquid isoparaffin, vaseline, squalene, scualane and the like are exemplified. In addition, natural oils such as camellia oil, macadamia nut oil, corn oil, olive oil, avocado oil, castor oil, safflower oil, jojoba oil, sunflower oil, canola oil, sesame oil, soybean oil, meadowfoam oil and the like; ester oils such as isopropyl myristate, isopropyl palmitate, myristyl myristate, octyl palmitate, stearyl stearate, isocetyl stearate, isononyl isononanoate, isotridecyl isononanoate, hydrogenated castor oil stearate, hydrogenated castor oil hydroxystearate, glyceryl tri(2-ethylhexanoate), pentaerythritol tetra(2-ethylhexanoate), neopentylglycol dicaprate, diglyceryl diisostearate, esters of dipentaerythritol with a mixed fatty acid such as hydroxystearic acid/stearic acid/rosin acids and the like, are exemplified.

The silicones, oil-based, or both components contained in the hair cosmetic composition of the present invention are preferably 0.1 to 15 mass %, and more preferably 0.5 to 10 mass %.

The typical aliphatic amines and their salts include, for example, the following compounds.

### (i) Alkylamide amines (and their salts)

For example, the compounds represented by the following general formula (12) and their salts are exemplified.

R²²-CONH-(CH₂)ₕ-N(R²³)₂ (12)

[In the formula, R²² represents an aliphatic hydrocarbon group having 11 to 23 carbon atoms, R²³ represents, same or different, an atomic hydrogen or alkyl group having 1 to 4 carbon atoms, and h represents an integer number of 2 to 4.]

One of the aliphatic amines and their salts contained in the hair cosmetic composition of the present invention may be employed, or two or more types thereof may be jointly employed, and in view of effects for providing enhanced softness and smoothness obtained by the use thereof, the content thereof is preferably 0.01 to 20 mass %, and more preferably 0.1 to 15 mass %, and even more preferably 0.5 to 10 mass %.

Further, the other components, which are generally employed in the hair cosmetic composition, may be blended to the hair cosmetic composition of the present invention in accordance with the purposes. For example, polymer compounds such as cationic cellulose, hydroxylated cellulose, highly-polymerized polyethylene oxide and the like; nonionic surfactants such as polyoxyethylene alkylethers, polyoxyethylene sorbitan fatty acid esters, glycerol fatty acid esters, polyglycerol fatty acid esters, polyoxyethylene hydrogenated castor oils, sucrose fatty acid esters, polyglycerol alkylethers, fatty acid alkanolamides, alkyl glycosides and the like; anti-dandruff agents such as zinc pyrithione, benzalkonium chloride and the like; vitamin compounds; bactericides; antiinflammatory agents; antiseptic agents; chelate agents; moisturizing agent such as panthenol and the like; coloring agents such as dye, pigment and the like; extracts such as eucalyptus extractants with polar solvent, proteins or their hydrolysates obtained from a shell having nacreous layer or a pearl, protein or their hydrolysate obtained from silk, protein-containing extractants obtained from seeds of legume, panax ginseng extractants rice germ extractants, fucus extractants, camellia extractants, aloe extractants, alpinia speciosa leaf extractants, chlorella extractant and the like; pearl powder such as titanium oxide and the like; flavors; coloring agents; ultraviolet absorbers; antioxidant agents; and other components described in [ENCYCLOPEDIA OF SHAMPOO INGREDIENTS (MICELLEPRESS)], are exemplified.

The pH (at 25 degrees C) of the hair cosmetic composition of the present invention when diluted 20 times by mass with water is preferably 1 to 5. The pH within this range provides maintaining soft touch feeling, achieving voluminous look for the entire hair, and better rising of the hair roots around the top of the head. The pH is more preferably adjusted to 2 to 5, and even more preferably 3 to 4.5, in view of obtaining the effect of smooth texture of the natural hair ordinarily has. The pH is controlled by employing acidic compositions such as inorganic acids, organic acids and the like, and further, sodium hydroxide may also be jointly employed as a basic substance. Such inorganic acids and organic acids contain compositions, which are used for neutralizing amido amine included in the above-described components.

In addition to above, it is preferable that the cosmetic composition of the present embodiment does not to contain larger amount of an anionic surfactant, in view of maintaining the soft touch. The content of the anionic surfactant is preferably equal to or lower than 1.0 mass %, more preferably equal to or lower than 0.1 mass %, and even more preferably equal to or lower than 0.05 mass %. It is even more preferable to contain substantially no anionic surfactant.

The hair cosmetic composition of the present invention is produced by dissolving the components (A) to (D) and other optional components in a solvent of water and components, for example, ethanol, 2-propanol, glycerol, propyleneglycol and the like as required. Typical composition forms include shampoo, hair rinse, hair conditioner, hair treatment, hair cream, hair pack and the like.

It is apparent that the present invention is not limited to the above embodiments, and may be modified and changed without departing from the scope and the spirit of the invention.

### EXAMPLES

The present invention will be further specifically described by referring to the following examples, though it is not intended to limit the scope of the present invention to these examples.

### Examples 1 to 8 and Comparative Examples 1 to 5

Hair cosmetic compositions having compositions shown in Tables 1 and 2 were produced by mixing and dissolving the respective components with heat and adding the dissolved components to water and then stirring the composition.

### Evaluation on the Touch

20 g of hair strands (about 15 to 20 cm) of hair, which were provided by Japanese women and were treated with cosmetic treatments such as cold-waving, hair-bleaching and the like, were tied together, and was cleaned with the shampoo. This hair was uniformly applied with 2 g of the hair cosmetic composition, and subsequently was rinsed off with running water for 30 seconds, and then towel drying was conducted, and further drying was conducted with a hair dryer, and sensory evaluations of the resultant hair conducted for: "smoothness after the rinsing off"; "voluminous look of the whole hair after the drying"; "absence of loosen hair/untidy hair after the drying"; "fingercomb smoothness after drying"; and "manageability after drying". The evaluations were carried out by five examinees on one-to-four scales on the basis of the following criteria for the evaluations, the total of the evaluations were shown in Tables 1 and 2.

Criteria for the evaluations
"Fingercomb smoothness after rinsing off"
4: significantly good fingercomb smoothness
3: slightly good fingercomb smoothness
2: not very good fingercomb smoothness
1: no good fingercomb smoothness

"Smoothness of the hair after towel drying"
4: significantly smooth
3: slightly smooth
2: not very smooth
1: no smooth

"Voluminous look of the whole hair after drying"
4: significantly voluminous for the entire hair
3: slightly voluminous for the entire hair
2: not very voluminous hair for the entire hair
1: no voluminous for the entire hair

"Absence of loosen hair/untidy hair after drying"
4: no appearance of loosen hair/untidy hair
3: few appearance of loosen hair/untidy hair
2: slight appearance of loosen hair/untidy hair
1: no appearance of loosen hair/untidy hair

"Manageability after drying"
4: significantly manageable
3: slightly manageable
2: not very manageable
1: no manageable

### "Easiness for the thermal shaping"

Commercially available curling iron (curling diameter of 3 cm) was employed to conduct two consecutive curling operations for the hair bundle after the sensory evaluation at a temperature of 130 to 150 degrees C for 5 seconds. The easiness for providing the shaping of the hair were evaluated by five examinees on one-to-four scales on the basis of the following criteria for the evaluations, The total of the evaluations were shown in Tables 1 and 2.

Criterion for the evaluation
"Easiness of thermal shaping"
4: significantly easy shaping
3: slightly easy shaping
2: not very easy shaping
1: no shaping

### "Rising level of the roots of the hair after drying"

Evaluations for the rising level of the hair were conducted as follows.

### <Preparation of model hair bundle>

The following materials were employed.
· Sample hair strands: 21 strands of hair of European blonds (length: 22 to 23 cm, mean diameter: 78.2 µm (n = 15; measured with a laser outer diameter measuring device) commercially available from DEMEOBROTHERS Inc.).
· Silicon micro tube: inner diameter × outer diameter (mm): 0.4 x 0.5, length: 1.5 cm x 7 tubes (part number: 1-8194-04, As-one Co., Ltd.)
· Steel wire (or stainless steel wire): diameter: 1.5 mm, length: 2.0 cm x 7 wires
· Cloth adhesive tape: 1 cm x 3 cm (NICHIBAN, No. 102 N)
· Vinyl tape: 0.6 cm x 1.0 cm (YAMATO Co., Ltd. NO200-19-5)
· Nylon fishing gut: diameter: 0.18 mm proper amount (HARISU LEICA HONPO)

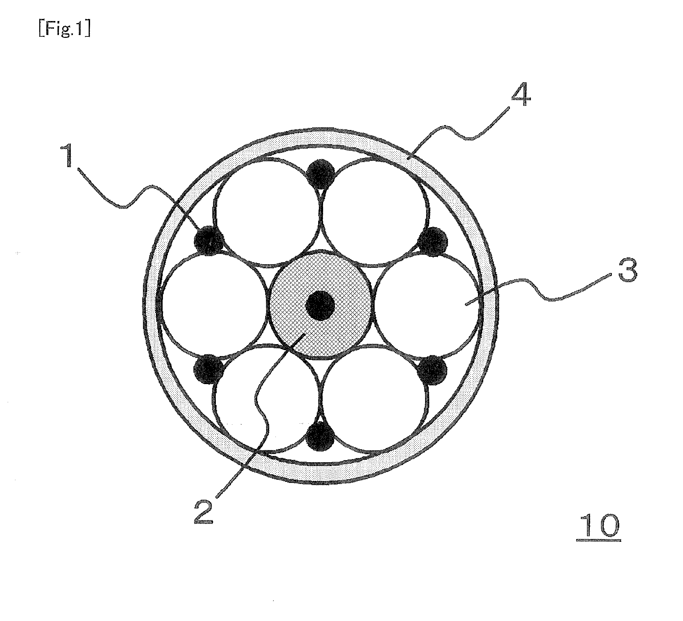

Fig. 1 is a diagram, which schematically illustrates a cross section of the model hair bundle 10. The above-described materials were employed to prepare the model hair bundle 10 shown in Fig. 1.
First, three strands of the sample hair aligned in the root direction were laced through a silicon tube of a diameter of 0.5 mm (inner diameter 0.4 mm) prepared into a segment having a length of 1.5 cm from the root site, and were fastened together with the silicon tube by creating a flat knot of the end of the sample to prepare the silicon tube 1. 7 sets of the silicon tube 1 were prepared in the similar manner. One set of the silicon tube 1 of these sets was wound with a vinyl tape 2 to increase its outer diameter to prepare a core section. Next, steel wires 3 (or stainless steel wires) having a length of 2 cm and a diameter of 1.5 mm were prepared, and each of the other six sets of the silicon tube 1 were arranged in the intervals among the six steel wires 3, and the core section and the other one steel wire 3 were bundled in the center, and the whole article was wound with the cloth adhesive tape 4. Further, this was wound with the gut 5 tightly over the cloth adhesive tape 4 to prepare the model hair bundle 10.

### <Evaluation method>

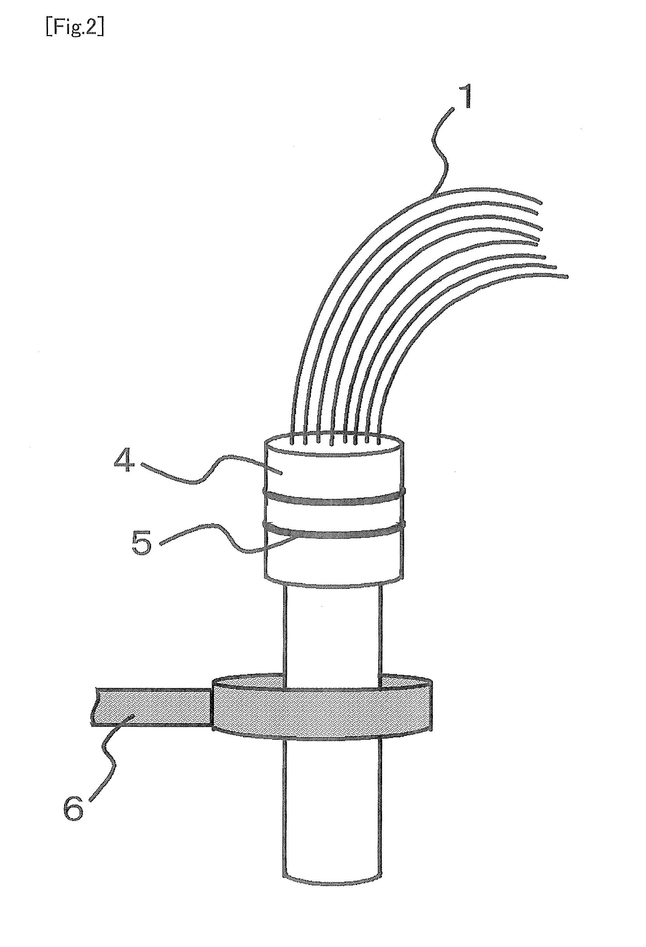

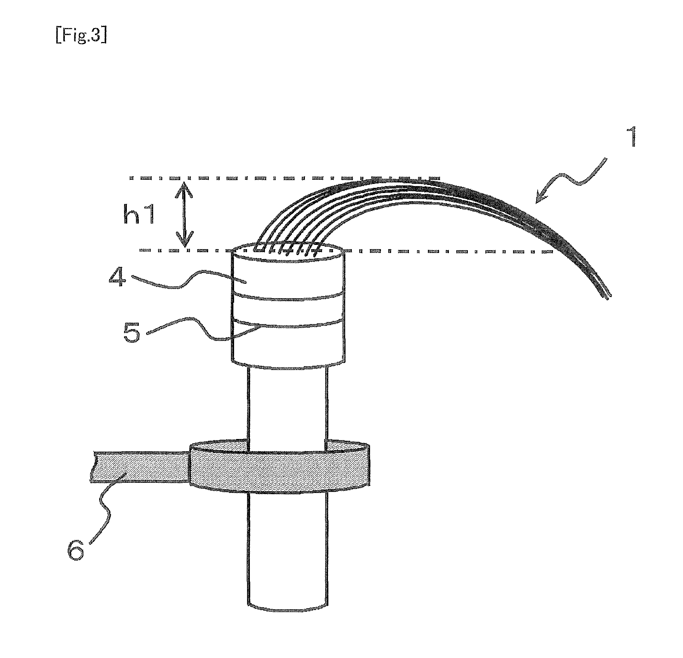

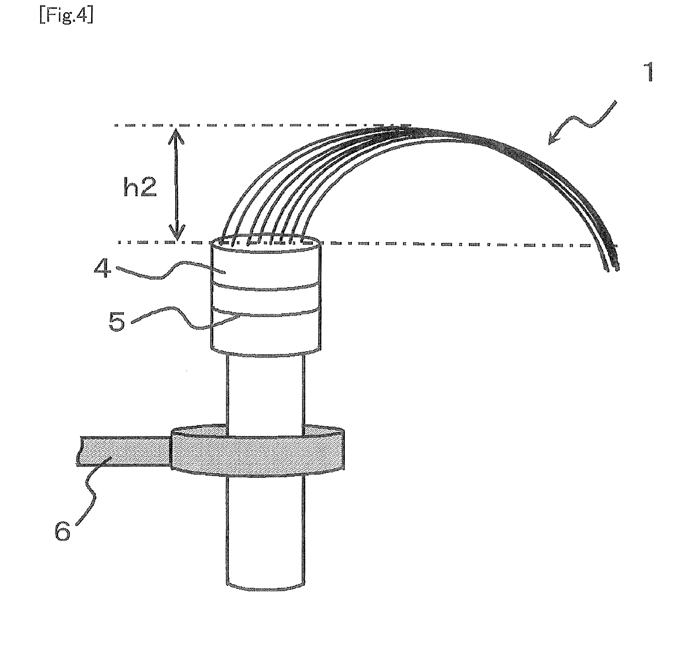

The method for measuring the rising level will be described in reference to Figs. 2 to 4.
The hair cosmetic composition was diluted to 20 times with ion-exchange water to obtain a sample aqueous solution.
In a manner represented by Fig. 2, the model hair bundle 10 was fixed by using a fixing tool 6, so as to be oriented upward, and 5 ml of the sample aqueous solution was dropped around an apex of a curvature section of the hair bundle of the sample hair, and then similar dropping operation was conducted with 5 ml of ion-exchange water to achieve sufficient rinsing, and then was sufficiently naturally dried by leaving it in an atmosphere at a room temperature (25 degrees C) and in a humidity of 50 (+/- 10) RH % for 10 minutes. The conditions of the curvature of the hair bundle before and after drying were taken with a digital camera, and the changes were recorded.
As shown in Fig. 3, the maximum value of the height of the curvature in the hair bundle just after the rinsing was defined as a height (h1 mm), and as shown in Fig. 4, the maximum value of the height of curvature in hair bundle after drying was defined as a height (h2, mm), and the rising level was determined as (Δh (mm) = h2 - h1).

[Table 1]

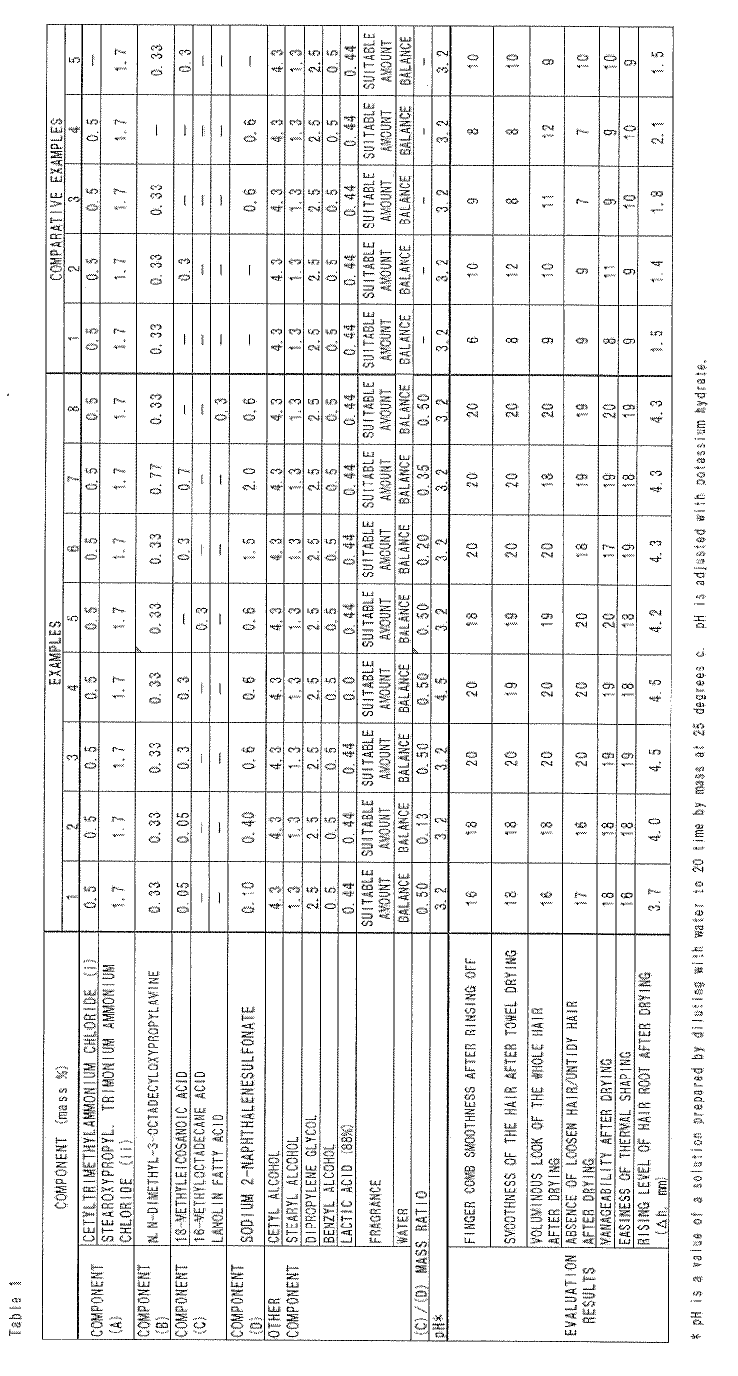

[Table 2]

**Table 2**

| COMPONENT (mass %) | | COMPARATIVE EXAMPLES | | |
|---|---|---|---|---|
| | | 6 | 7 | 8 |
| COMPONENT (A) | CETYLTRIMETHYLAMMONIUM CHLORIDE (i) | 0. 5 | 0.5 | 0. 5 |
| | STEAROXYPROPYL. TRIMONIUM AMMONIUM CHLORIDE (ii) | 1. 7 | 1. 7 | 1. 7 |
| COMPONENT (B) | N, N-DIMETHYL-3-OCTADECYLOXYPROPYLAMINE | 0. 33 | 0. 33 | 0. 33 |
| COMPONENT (C) | 18-METHYLEICOSANOIC ACID | - | - | 0. 3 |
| | ISOSTEARIN T (ISOOCTADECANE) (COMMERCIALLY AVAILABLE FROM NISSAN CHEMICAL INDUSTRIES, LTD.) | 0.3 | - | - |
| COMPONENT (D) | SODIUM 2-NAPHTHALENESULFONATE | 0.6 | 0.6 | - |
| | SODIUM BUTANESULFONATE | - | - | 0.6 |
| OTHER COMPONENT | CETYL ALCOHOL | 4.3 | 4. 3 | 4.3 |
| | STEARYL ALCOHOL | 1.3 | 1. 3 | 1.3 |
| | DIPROPYLENE GLYCOL | 2. 5 | 2.5 | 2. 5 |
| | BENZYL ALCOHOL | 0. 5 | 0.5 | 0. 5 |
| | LACTIC ACID (88%) | 0.44 | 0.44 | 0.44 |
| | FRAGRANCE | SUITABLE AMOUNT | SUITABLE AMOUNT | SUITABLE AMOUNT |
| | WATER | BALANCE | BALANCE | BALANCE |
| (C)/(D) MASS RATIO | | - | - | - |
| pH* | | 3. 2 | 3. 2 | 3.2 |
| EVALUATION RESULTS | FINGER COMB SMOOTHNESS AFTER RINGING OFF | 5 | 9 | 8 |
| | SMOOTHNESS OF THE HAIR AFTER TOWEL DRYING | 5 | 8 | 8 |
| | VOLUMINOUS LOOK OF THE WHOLE HAIR AFTER DRYING | 5 | 11 | 7 |
| | ABSENCE OF LOOSEN HAIR/UNTIDY HAIR AFTER DRYING | 9 | 7 | 9 |
| | MANAGEAEILITY AFTER DRYING | 8 | 9 | 8 |
| | EASINESS OF THERMAL SHAPING | 7 | 10 | 6 |
| | RISING LEVEL OF HAIR ROOT AFTER DRYING (Δh, mm) | 1. 1 | 1. 8 | 1. 1 |

| | | | | |
|---|---|---|---|---|
| * pH is a value of a solution prepared by diluting with water to 20 time by mass at 25 degrees c. pH is adjusted with potassium hydrate. | | | | |

## Claims

1. A hair cosmetic composition, containing:
(A) equal to or higher than 0.01 mass % and equal to or lower than 20 mass % of a quaternary ammonium represented by the following formula (1) or its salt;
R¹R²R³R⁴N⁺X⁻ (1)
[In the formula (1), at least one of R¹, R², R³ and R⁴ represents a linear or branched alkyl group or alkenyl group having 16 to 25 carbon atoms, which may have an ether bond, at least two or more of the rest thereof represent a same or different alkyl group having 1 to 6 carbon atoms or -(AO)ₘH (A represents a same or different an alkylene group having 2 to 4 carbon atoms, m represents an integer number of 1 to 6, and the sequence is arbitrary) and X⁻ represents a halide ion or alkyl sulfate ion having 1 to 2 carbon atoms.]
(B) equal to or higher than 0.01 mass % and equal to or lower than 20 mass % of ether-type tertiary amine, represented by the following formula (2) or its salt;
R⁹-(O-R²⁴)ₑ-NR¹⁰R¹¹ (2)
[In the formula (2), R⁹ represents a linear or branched alkyl group or alkenyl group having 6 to 24 carbon atoms; R¹⁰ and R¹¹ represent a same or different alkyl group having 1 to 6 carbon atoms or -(AO)ₘH (A represents a same or different alkylene group having 2 to 4 carbon atoms, m represents an integer number of 1 to 6, and the sequence is arbitrary) and R²⁴ represents a linear alkyl group having 1 to 3 carbon atoms, which may be substituted with hydroxy group (e represents an integer number of 1 to 5).]
(C) equal to or higher than 0.005 mass % and equal to or lower than mass % of branched aliphatic acid represented by the following formula (3) or its salt;
CH₃CHR¹⁸- (CH₂)ₙCOOH (3)
[In formula (3), R¹⁸ represents a methyl group or ethyl group and n represents an integer number of 14 to 20.], and
(D) equal to or higher than 0.05 mass % and equal to or lower than 10 mass % of aromatic sulfonate; and water,
wherein the mass ratio of the component (C) and the component (D) being (C)/(D) is 0.05 to 1.

2. The hair cosmetic composition according to claim 1, wherein the component (A) contains trimethylammonium having an alkyl group having 16 to 22 carbon atoms or its salt.

3. The hair cosmetic composition according to claim 2, wherein the component (A) at least contains cetyltrimethylammonium or its salts.

4. The hair cosmetic composition according to any one of claims 1 to 3, wherein said composition contains the component (B), in which R⁹ in said formula (2) contains a linear or branched alkyl group or alkenyl group having 14 to 22 carbon atoms.

5. The hair cosmetic composition according to any one of claims 1 to 3, wherein the component (B) contains one, two or more compound(s) selected from the group consisting of N,N-dimethyl-3-hexadecyloxypropylamine, N,N-dimethyl-3-octadecyloxypropylamine, and their salts.

6. The hair cosmetic composition according to any one of claims 1 to 5, wherein said composition contains the component (C), in which n in said formula (3) represents an integer number of 16 to 20.

7. The hair cosmetic composition according to any one of claims 1 to 5, wherein the component (C) contains one, two or more compound(s) selected from the group consisting of 18-methyleicosanoic acid, 14-methylpentadecanoic acid, 14-methylhexadecanoic acid, 15-methylhexadecanoic acid, 15-methylheptadecane acid, 16-methylheptadecane acid, 16-methyloctadecane acid, 17-methyloctadecane acid, 17-methylnonadecane acid and lanolin fatty acid.

8. The hair cosmetic composition according to any one of claims 1 to 7, wherein the component (D) at least contains 2-naphthalenesulfonate.

9. The hair cosmetic composition according to any one of claims 1 to 8, wherein the mass ratio of the component (C) and the component (D) being (C)/(D) is 0.1 to 5.

10. The hair cosmetic composition according to any one of claims 1 to 9, wherein pH (at 25 degrees C) of a solution of said hair cosmetic composition diluted to 20 times by mass with water is 1 to 5.

11. The hair cosmetic composition according to claim 10, wherein pH (at 25 degrees C) of a solution of said hair cosmetic composition diluted to 20 times by mass with water is 3 to 4.5.

12. The hair cosmetic composition according to any one of claims 1 to 11, wherein a content of an anionic surfactant over the whole hair cosmetic composition is equal to or lower than 1.0 mass %.

13. A use of said hair cosmetic composition according to any one of claims 1 to 12, wherein said hair cosmetic composition is applied to the hair and then is rinsed with water to provide a conditioning for the hair.

14. A use of said hair cosmetic composition according to any one of claims 1 to 12 for producing a product, which is applied to the hair and then is rinsed with water to provide conditioning for the hair.

15. A method including applying said hair cosmetic composition according to any one of claims 1 to 12 to the hair and then is rinsed with water.
